# FASCICULE DE BREVET EUROPEEN

(11) **EP 4 590 176 B1**
(45) Date de publication et mention de la délivrance du brevet: **09.12.2026**
(21) Numéro de dépôt: 23745615.7
(22) Date de dépôt: 03.08.2023
(51) Int. Cl.: A61B 5/00, A61B 5/01

(54) **HOUSSE MULTICOUCHES POUR MAINTENIR UN DISPOSITIF SUR UN CORPS HUMAIN**
MEHRSCHICHTIGER ÜBERZUG ZUR HALTERUNG EINER VORRICHTUNG AN EINEM MENSCHLICHEN KÖRPER
MULTILAYER COVER FOR HOLDING A DEVICE ON A HUMAN BODY

(30) Priorité: 19.09.2022 FR 2209449
(43) Date de publication de la demande: 30.07.2025
(73) Titulaire: F2D Medical, 14460 Colombelles (FR)
(72) Inventeur: D'ESTAIS, Mathias, 14000 CAEN (FR); MENARD, Benjamin, 14280 AUTHIE (FR); VAUPRES, Maxime, 14190 Urville (FR)
(74) Mandataire: IPAZ
(86) Numéro de dépôt international: PCT/EP2023/071530
(87) Numéro de publication internationale: WO 2024/061524

(56) Documents cités:
- FR-A1- 3 097 115
- US-A- 5 566 671
- US-A1- 2009 254 053
- US-A1- 2011 144 470

## Description

### Domaine technique

L'invention se rapporte au domaine du maintien d'un dispositif sur le corps d'un individu. De manière non limitative, ce dispositif peut être un dispositif médical et/ou de mesure de données physiologiques et/ou de diffusion d'un principe actif sous forme liquide et/ou de prélèvement d'un fluide corporel.

A titre d'exemple, le dispositif peut comporter des composants électroniques tels que des capteurs ou des antennes, des moyens de communication par voie filaire ou sans fil, des moyens d'enregistrement et de mémorisation de données, des équipements électromécaniques tels que des micro-pompes, des micro-aiguilles ou encore des vibreurs, des organes agissant sur le corps tels que des diffuseurs d'actifs pharmacologiques, avec microaiguilles ou en patch, ou des aimants, des moyens de prélèvement de fluide du type sang ou transpiration, des moyens de diagnostic in vitro, etc.

L'invention présente un intérêt particulier, nullement limitatif, dans le secteur de la donnée patient d'aide au diagnostic, le dispositif pouvant en particulier être un dispositif médical de thermométrie prévu pour mesurer la température corporelle de manière continue pendant une période de plusieurs heures ou jours. A ce titre, il peut aussi trouver des applications dans les secteurs de la chronothérapeutique et de la chronopharmacologie.

### État de la technique antérieure

Une première technique de maintien de dispositif, connue dans l'état de la technique antérieure, consiste à coller le dispositif sur la peau à l'aide d'un ruban adhésif à double face. Entre autres inconvénients, la colle du ruban adhésif peut souiller le dispositif ce qui nécessite de le nettoyer en cas de réutilisation. De plus, ce mode de fixation ne permet pas de maintenir le dispositif collé lorsqu'un effort relativement important est exercé sur le dispositif. Cela peut notamment faciliter l'arrachement du dispositif sous l'action d'un mouvement de l'individu dans un lit.

Une deuxième technique connue consiste à maintenir le dispositif à l'aide d'un film adhésif, en silicone ou en polyuréthane, en choisissant un film de surface généreuse, plus large et plus long que le dispositif de manière à recouvrir largement le dispositif. Le dispositif est typiquement disposé au niveau d'une partie centrale du film, lequel est collé sur la peau par des parties périphériques. En général, un tel film adhésif comprend de la colle à la fois sur les parties périphériques et sur la partie centrale, entraînant ainsi un souillage du dispositif.

Certains films adhésifs comprennent une couche de colle recouvrant uniquement des parties périphériques du film, ce qui permet d'éviter le dépôt de résidus de colle sur le dispositif. En pratique, un tel film est toutefois difficile à poser et tend à générer des plis et tensions ne permettant pas de maintenir correctement le dispositif, a fortiori lorsque celui-ci est relativement lourd et/ou volumineux. Pour améliorer la fixation, un tel film adhésif peut en outre nécessiter l'utilisation d'un type de colle et/ou d'une quantité de colle risquant d'augmenter l'agression de la peau.

Une troisième technique connue consiste à loger le dispositif dans une gaine élastique ou un brassard enserrant un membre tel qu'un bras. Les brassards connus sont généralement confectionnés en couture sur une base de tissu ou cuir ou d'élastomère. Ils sont susceptibles de bouger autour du membre ou en glissement le long du membre, ne permettant pas de maintenir le dispositif dans une position précise sur le corps. A fortiori lorsque le dispositif est relativement lourd et/ou volumineux, il peut être nécessaire de réaliser un serrage important du brassard ou de la gaine autour du membre, rendant le portage pénible voire insupportable pour l'individu. Ce type de technologie n'est par ailleurs pas compatible avec un maintien du dispositif sur des parties du corps telles que le torse ou un flanc. Enfin, ce type de technologie est difficilement industrialisable et nécessite un nettoyage avant réutilisation.

Les documents US5566671A, US2009/254053A1 et US2011/144470A1 divulguent des structures prévues pour maintenir des dispositifs ou composants médicaux sur la peau de patients.

### Exposé de l'invention

L'invention vise à procurer une solution permettant de maintenir un dispositif sur un corps de manière stable, précise, durable et confortable, tout en remédiant aux inconvénients précités.

Un but particulier de l'invention est de procurer une solution permettant de réduire les coûts de fabrication.

Un autre but de l'invention est de procurer un système de maintien permettant de simplifier la pose de ce système et du dispositif qu'il est destiné à maintenir, ainsi que les différentes étapes associées à l'utilisation de ce système, notamment en milieu hospitalier, notamment la mise en marche du dispositif pour le cas d'un dispositif actif, le contrôle visuel de son état de fonctionnement, le contrôle visuel de son bon positionnement, l'extraction du dispositif de la housse et/ou son retrait de la housse.

A cet effet, l'invention a pour objet une housse telle que définie dans la revendication 1, destinée à maintenir un dispositif tel qu'un dispositif médical et/ou de mesure de données physiologiques et/ou de diffusion d'un principe actif, par exemple sous forme liquide, et/ou de prélèvement d'un fluide corporel à proximité de, ou contre, une surface de peau d'un individu.

L'invention procure ainsi une housse qui présente une configuration globale relativement simple, à savoir deux couches initialement plaquées l'une contre l'autre, tout en permettant d'obtenir un maintien en position du dispositif qui soit stable, précis et durable.

La structure en couches permet d'une part de simplifier et réduire les coûts de fabrication de la housse et, d'autre part, de rendre celle-ci jetable afin d'améliorer le respect des normes hygiéniques et réduire les contraintes de gestion, notamment en milieu hospitalier.

La housse de l'invention peut notamment être utilisée pour maintenir un dispositif contre ou à proximité d'une surface de peau de différentes parties du corps, par exemple une partie telle que le torse présentant un rayon de courbure important, ou telle qu'un bras ou un autre membre.

Ledit espace de réception ainsi que ladite ouverture permettent de loger le dispositif dans la housse de manière amovible et de le maintenir en position sans collage, ce qui permet à la fois de ne pas souiller le dispositif et de réduire le risque d'arrachement.

Dans un mode de réalisation, la première couche et la deuxième couche comprennent chacune une surface interne et sont reliées l'une à l'autre par collage ou soudage de parties de leur surface interne.

Dans un mode de réalisation, la première couche et/ou la deuxième couche comprennent un matériau nontissé.

Le terme « nontissé » s'entend ici dans une acception très large et inclut tous les matériaux légers et fins qui peuvent être fournis en bobine.

Ces matériaux ont l'avantage d'être généralement peu coûteux et aussi d'être transformables dans un procédé en ligne permettant d'obtenir un produit fonctionnellement intéressant tout en étant suffisamment optimisé économiquement pour être jetable.

Le matériau de prédilection est le nontissé au sens propre, c'est-à-dire selon la définition ISO 9092, un matériau constitué de fibres orientées de manière aléatoire ou directionnelle, consolidées et liées par friction, et/ou cohésion et/ou adhésion.

Les fibres utilisées peuvent être naturelles telles que de la laine, du chanvre, du coton, de la cellulose, des fibres minérales telles que la fibre de verre ou céramique, mais dans l'industrie on trouve essentiellement des nontissés dont les fibres sont en matière plastiques, avec de nombreuses variantes : des fibres de polypropylène, de polyéthylène, polyester, chlorure de polyvinyle, etc.

Le papier est un autre matériau inclus dans la définition de nontissé dans le présent document. Bien que les liaisons entre les fibres de cellulose diffèrent entre les procédés de production de papier et les procédés de nontissé au sens propre, le procédé est très proche et son comportement peut rentrer dans la fabrication de couches selon l'invention.

De même, les films coulés, soufflés ou extrudés à partir de polymères sont aussi inclus dans la définition de nontissés selon l'invention car leur comportement peut rentrer dans la fabrication de certaines couches selon l'invention. On note en particulier dans cette catégorie l'existence de films élastomère pouvant conférer une certaine élasticité au matériau pouvant être intéressante, notamment pour ladite première couche.

Enfin, certains textiles légers peuvent rentrer fonctionnellement dans la conception de la housse de l'invention, et aussi être compatibles avec les objectifs de prix. On note en particulier la possibilité d'utiliser des voiles tissés ou des tissus légers. Ils peuvent être utilisés pour la première couche et la deuxième couche mais ils ont généralement une main faible et seront donc utilisés de manière privilégiée pour la deuxième couche plutôt que pour la première couche. Ils peuvent aussi avoir des caractéristiques d'élasticité intéressantes, ce qui est particulièrement intéressant pour la deuxième couche.

Dans un mode de réalisation, la première couche et/ou la deuxième couche peuvent comprendre un matériau nontissé, au sens de la norme ISO 9092, ou bien un matériau plastique ou élastomère.

Dans un mode de réalisation préféré, la première couche comprend un matériau nontissé, au sens de la norme ISO 9092, et la deuxième couche comprend un matériau plastique.

En variante, la première couche et/ou la deuxième couche peuvent comprendre un textile.

Plus généralement, la première couche et la deuxième couche peuvent être réalisées dans un même matériau ou dans un matériau différent l'une par rapport à l'autre. Ainsi, à titre d'exemple non limitatif, la première couche peut comprendre un premier matériau tel qu'un matériau textile et la deuxième couche peut comprendre un deuxième matériau tel qu'un matériau élastomère. Pour autre exemple, la première couche et la deuxième couche peuvent toutes deux comprendre un matériau plastique, par exemple du polypropylène pour la première couche et du polyuréthane pour la deuxième couche.

L'utilisation de matériaux nontissés, dans l'acception large définie ci-dessus, permet notamment de fabriquer une housse légère, économique, compatible avec un procédé de fabrication par déroulement de bobines de matière, du type « rouleau à rouleau » (voir plus loin ci-dessous), de simplifier le collage / soudage des différentes couches et d'accéder ainsi à un prix de revient compatible avec le fait de considérer la housse comme jetable.

Dans un mode de réalisation, la deuxième couche est extensible ou élastique.

Bien entendu, la première couche peut aussi être extensible ou élastique.

Indépendamment du matériau respectif de la première couche et de la deuxième couche, il est préféré que la housse autorise une déformation plastique ou élastique de la deuxième couche plus importante que la première couche.

Une telle caractéristique permet notamment de réduire le risque de formation de plis dans la première couche et d'améliorer la tenue de la housse sur la surface de peau.

La housse de l'invention peut présenter de nombreuses caractéristiques dimensionnelles et géométriques.

De manière non limitative, la première couche peut avoir une épaisseur supérieure, par exemple plus de deux fois supérieure, à une épaisseur de la deuxième couche.

Selon l'invention, la première couche a un coefficient de drapé supérieur à celui de la deuxième couche.

La première couche peut par exemple avoir un coefficient de drapé plus de deux fois supérieur à celui de la deuxième couche et/ou une « main » (voir plus loin ci-dessous) supérieure à celle de la deuxième couche et/ou une résistance cylindrique (voir plus loin ci-dessous) supérieure, par exemple plus de deux fois supérieure, à celle de la deuxième couche.

De telles caractéristiques, a fortiori lorsqu'elles sont combinées entre elles et/ou avec la caractéristique précitée de déformation plastique / élastique relative des deux couches, permettent d'améliorer la tenue de la housse sur la surface de peau ainsi que le maintien en position du dispositif.

De manière non limitative, lorsque la housse est dans l'état initial, la première couche peut avoir une longueur supérieure à une largeur de cette première couche, de préférence supérieure à trois fois sa largeur. A titre indicatif, la longueur de la première couche peut être comprise entre 30 mm et 800 mm, de préférence entre 60 mm et 400 mm.

Dans un mode de réalisation, la première couche comprend une découpe autorisant un contact direct d'une partie du dispositif avec la surface de peau lorsque le dispositif est reçu dans l'espace de réception.

Une telle découpe autorise notamment un contact direct de parties ou éléments du dispositif, tels que des micro-aiguilles ou encore des capteurs optiques, avec la peau et/ou permet de ne pas bloquer la transpiration.

A titre indicatif, la découpe peut avoir une dimension allant jusqu'à 70 % de la surface totale de la première couche, tout en conservant néanmoins une zone de collage suffisante.

Dans un mode de réalisation, la housse comprend une troisième couche ayant une première partie reliée à la première couche et/ou à la deuxième couche et une deuxième partie configurée pour être collée sur ladite surface de peau et/ou sur une partie de la housse et/ou sur une partie du dispositif, par exemple de manière à former un brassard.

Dans un mode de réalisation, la première couche et/ou la deuxième couche comprennent une surface interne revêtue d'un matériau adjuvant tel qu'une huile, du polytétrafluoroéthylène, du talc ou encore une poudre de nanoparticules, de manière à réduire le glissement du dispositif lorsque celui-ci est reçu dans l'espace de réception de la housse.

L'invention a aussi pour objet un ensemble comprenant une housse telle que définie ci-dessus ainsi qu'un dispositif tel qu'un dispositif médical et/ou de mesure de données physiologiques et/ou de diffusion d'un principe actif et/ou de prélèvement d'un fluide corporel.

Dans un mode de réalisation, le dispositif est un dispositif médical de thermométrie, destiné par exemple à mesurer la température corporelle de manière continue pendant une période de plusieurs heures ou jours.

Plus généralement, le dispositif peut intégrer des composants électroniques tels que des capteurs ou des antennes, des moyens de communication par voie filaire ou sans fil, des moyens d'enregistrement et de mémorisation de données, des équipements électromécaniques tels que des micro-pompes, des micro-aiguilles ou encore des vibreurs, des organes agissant sur le corps tels que des diffuseurs d'actifs pharmacologiques, avec microaiguilles ou en patch, ou des aimants, des moyens de diagnostic in vitro, des moyens de captation de données et/ou d'analyse de fluides corporels, etc.

L'invention a aussi pour objet un procédé de fabrication d'une housse telle que définie ci-dessus.

Le procédé comprend de préférence une étape de déroulement d'une ou plusieurs bobines de film de manière à former la première couche et/ou la deuxième couche et une étape d'assemblage de la première couche avec la deuxième couche.

Un tel procédé, connu sous la dénomination « rouleau à rouleau » (« roll-to-roll » en anglais), permet de fabriquer des housses conformes à l'invention avec une cadence élevée, la vitesse de déroulement des bobines de film pouvant typiquement être de l'ordre d'un mètre par seconde.

Un tel procédé permet plus généralement de réduire les coûts de fabrication.

Dans un mode de réalisation, l'étape d'assemblage comprend un déroulement d'une ou plusieurs bobines d'un film adhésif, notamment un film adhésif double face.

Par ailleurs, l'invention a aussi pour objet un procédé de pose et/ou de fixation d'une housse telle que définie ci-dessus sur une surface de peau d'un individu.

Ce procédé comprend une étape d'application de la première couche sur la surface de peau.

Le procédé peut comprendre une étape d'insertion du dispositif dans l'espace de réception de la housse.

L'insertion est de préférence réalisée avant fixation de la housse sur la surface de peau, afin de faciliter l'insertion du dispositif dans la housse. Bien entendu, l'insertion du dispositif peut alternativement être réalisée après collage de la housse sur la surface de peau

D'autres avantages et caractéristiques de l'invention apparaîtront à la lecture de la description détaillée, non limitative, qui suit.

### Brève description des dessins

La description détaillée qui suit fait référence aux dessins annexés sur lesquels :
[Fig. 1] est une vue schématique, en perspective et en éclaté, d'une housse conforme à un premier mode de réalisation de l'invention ;
[Fig. 2] est une vue schématique de dessus d'une première couche de la housse de la figure 1 ;
[Fig. 3] est une vue schématique de dessus d'une deuxième couche de la housse de la housse de la figure 1 ;
[Fig. 4] est une vue schématique de dessus d'un dispositif destiné à être logé dans la housse de la figure 1;
[Fig. 5] est une vue schématique de dessous du dispositif de la figure 4 ;
[Fig. 6] est une vue schématique en coupe d'un cylindre de matière et d'un outillage permettant de tester la résistance du cylindre ;
[Fig. 7] est une vue schématique, en perspective et en éclaté, d'une housse conforme à un deuxième mode de réalisation de l'invention.

### Description détaillée de modes de réalisation

Les figures comprennent un référentiel définissant une direction longitudinale D1, une direction transversale D2 et une direction latérale D3 orthogonales entre elles.

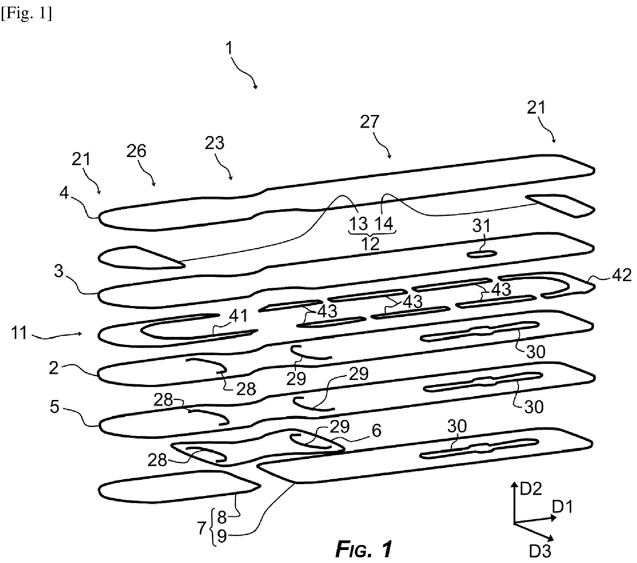

Il est représenté sur la figure 1, en éclaté, différentes couches formant une housse 1 conforme à l'invention.

Dans le présent document, une couche désigne une structure ayant une épaisseur faible par rapport à son étendue superficielle.

En référence à la figure 1, chacune des couches de la housse 1 comporte deux surfaces espacées le long de la direction D2, l'une de ces surfaces, appelée « première surface », étant visible sur la figure 1, l'autre surface de chacune des couches, appelée « deuxième surface », n'étant pas visible sur la figure 1. Pour chacune des couches, la distance selon D2 entre la première surface et la deuxième surface définit l'épaisseur de cette couche.

Dans ce mode de réalisation, qui n'est pas limitatif, la housse 1 comprend plus spécifiquement les couches référencées 2 à 7, 11 et 12 qui sont empilées le long de la direction D2.

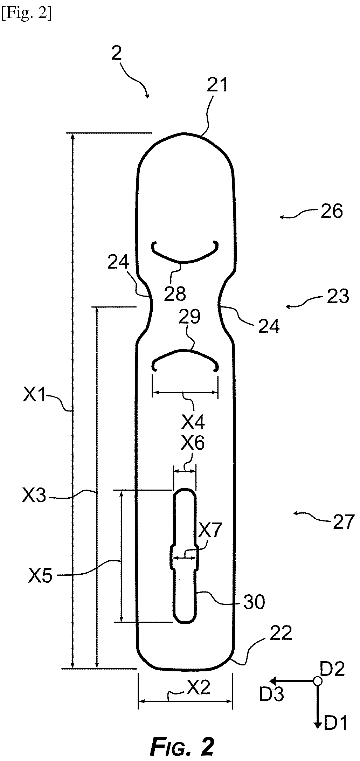

En référence à la figure 2 qui montre la couche 2 séparément, cette couche 2 présente une forme globalement rectangulaire et allongée le long de la direction D1.

Dans cet exemple particulier, la couche 2 comprend un nontissé de polypropylène et présente une longueur X1 de 280 mm, une largeur X2 de 50 mm et une épaisseur de 410 µm.

La couche 2 présente deux extrémités longitudinales 21 et 22 le long de D1. Dans cet exemple, l'extrémité 21 forme un demi-cercle ayant un rayon correspondant à la moitié de la largeur X2, soit en l'occurrence 25 mm, constituant une extrémité 21 arrondie. L'extrémité 22 a quant à elle des coins arrondis qui, dans cet exemple, présentent chacun un rayon de 15 mm.

La largeur de la couche 2 est sensiblement constante le long de D1, entre les extrémités 21 et 22, à l'exception d'une région 23 de la couche 2 ayant une largeur réduite. Cette réduction de largeur est réalisée sous forme de découpes 24 en demi-lunes, positionnées à une distance longitudinale X3 de l'extrémité 22. Dans cet exemple, X3 est environ égale à 190 mm.

La couche 2 comprend ainsi deux parties 26 et 27 qui s'étendent longitudinalement de part et d'autre de la région 23.

La couche 2 comprend par ailleurs deux ouvertures 28 et 29 réalisées respectivement dans les parties 26 et 27, longitudinalement à proximité de la région 23.

Les ouvertures 28 et 29 sont réalisées sous forme de fentes s'étendant latéralement sur une grande partie de la largeur de la couche 2, de manière à présenter une dimension latérale X4 qui correspond sensiblement à la largeur de la région 23. Dans cet exemple, X4 est environ égale à 33 mm.

La partie 27 de la couche 2 comprend aussi une découpe 30 oblongue s'étendant longitudinalement. Dans cet exemple, la découpe 30 a une longueur X5 d'environ 70 mm et une largeur X6 d'environ 11 mm sur l'essentiel de la longueur de cette découpe 30, la découpe 30 ayant une partie centrale présentant une largeur X7 légèrement supérieure, dans cet exemple une largeur X7 d'environ 13 mm.

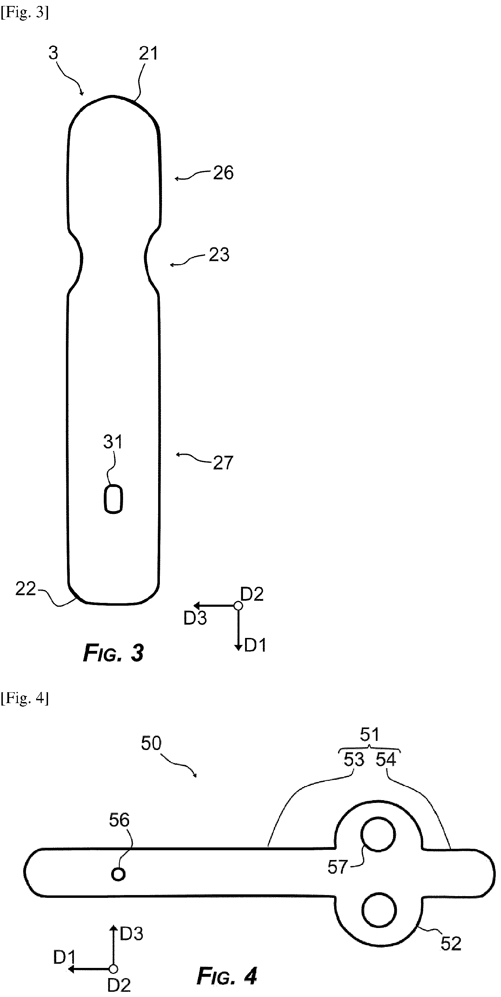

En référence à la figure 3, la couche 3 présente une géométrie globale identique à celle de la couche 2. La couche 3 est décrite ci-après uniquement selon ses différences par rapport à la couche 2, la description qui précède s'appliquant par analogie.

Dans cet exemple particulier, la couche 3 est réalisée en polyuréthane et a une épaisseur de 30 µm.

Ce matériau permet de conférer à la couche 3 des propriétés élastiques.

La couche 3 ne comprend pas des ouvertures telles que les ouvertures 28 et 29 de la couche 2, ni une découpe telle que la découpe oblongue 30 de la couche 2.

La partie 27 de la couche 3 comprend cependant une découpe 31 dont la position et les dimensions correspondent sensiblement à celles de ladite partie centrale de la découpe 30 de la couche 2.

En référence à la figure 1, la couche 11 est une couche de film de colle double-face ayant subie des découpes au cours du procédé afin de former différentes parties configurées pour relier les couches 2 et 3 l'une à l'autre.

Plus précisément, les différentes parties de la couche de colle 11 sont agencées de manière à être disposées sur une partie périphérique de la première surface de la couche 2 et sur une partie périphérique correspondante de la deuxième surface de la couche 3.

La partie 26 des couches 2 et 3 ainsi assemblées forme un premier espace qui est en l'occurrence délimité :
- transversalement, d'une part, par une partie de la première surface de la couche 2 et, d'autre part, par une partie de la deuxième surface de la couche 3, la première surface de la couche 2 et la deuxième surface de la couche 3 formant ainsi des surfaces internes,
- longitudinalement du côté de l'extrémité longitudinale 21, par une partie 41 de la couche de colle 11,
- latéralement, par des portions de la partie 41 de la couche de colle 11.

De manière semblable, la partie 27 des couches 2 et 3 ainsi assemblées forme un deuxième espace délimité :
- transversalement, par une partie des surfaces internes des couches 2 et 3,
- longitudinalement du côté de l'extrémité longitudinale 22, par une partie 42 de la couche de colle 11,
- latéralement, par des portions de la partie 42 et par d'autres parties 43 de la couche de colle 11.

Lorsque la housse 1 est dans un état initial, les couches 2 et 3 sont plaquées l'une contre l'autre de sorte que le premier espace et le deuxième espace qu'elles forment ont une dimension selon D2, ou épaisseur, très faible ou nulle par endroits. Dans cet exemple, à l'état initial, l'intégralité de la surface interne de la couche 3 est disposée en regard de la surface interne de la couche 2 et réciproquement.

La couche 3 est susceptible d'être déformée de manière plus importante que la couche 2, compte tenu notamment de l'épaisseur respective de ces couches et de leur matériau respectif. En l'occurrence, la déformabilité de la couche 2 est négligeable par rapport à la couche 3.

Il va maintenant être décrit la structure des autres couches de la housse 1 de la figure 1.

La couche 5 présente une géométrie globale identique à celle de la couche 2. La couche 5 est décrite ci-après uniquement selon ses différences par rapport à la couche 2, la description précédente de la figure 2 s'appliquant par analogie.

Dans cet exemple, la couche 5 est une couche de colle en silicone ayant une épaisseur d'environ 180 µm.

La couche de colle 5 est disposée contre, et donc collée à, la deuxième surface de la couche 2 qui forme une surface externe de la couche 2, de manière à constituer un revêtement adhésif.

La couche 6 présente une géométrie globalement identique à une partie de la couche 2, cette partie intégrant la région 23 de la couche 2 et une portion des parties 26 et 27 comportant les ouvertures 28 et 29 (voir figures 1 et 2).

Dans cet exemple particulier, la couche 6 est réalisée en polyéthylène téréphtalate et a une épaisseur d'environ 23 µm.

La couche 6 est disposée contre, et donc collée à, la deuxième surface de la couche de colle 5 de manière à faire coïncider les ouvertures 28 et 29 réalisées dans chacune des couches 2, 5 et 6.

La couche 6 forme ainsi un revêtement permettant de constituer une zone non adhésive longitudinalement au niveau de la région 23 et des ouvertures 28 et 29 de la couche 2. Contrairement à la couche 7 présentée ci-après qui est un protecteur pelable, la couche 6 est destinée à être laissée sur la housse lors de l'usage.

La couche 7 comprend deux parties 8 et 9 présentant une géométrie globale identique aux parties 26 et 27 de la couche 2.

Dans cet exemple, chacune des parties 8 et 9 de la couche 7 est réalisée en polyéthylène à basse densité et a une épaisseur d'environ 50 µm.

La partie 9 de la couche 7 comprend une découpe identique à la découpe oblongue 30 de la couche 2. La couche 7 ne comprend toutefois pas d'ouvertures telles que les ouvertures 28 et 29 de la couche 2

La couche 7 est disposée contre la deuxième surface de la couche de colle 5 et de la couche 6, entraînant un collage de la première surface de la partie 8 de la couche 7 sur une partie de la deuxième surface de la partie 26 de la couche de colle 5 et un collage de la première surface de la partie 9 de la couche 7 sur une partie de la deuxième surface de la partie 27 de la couche de colle 5.

La couche 7 forme un film de protection destiné à être retiré pour coller la housse 1 lors de son utilisation (voir plus loin ci-dessous).

Lorsque les couches 2, 3, 5, 6, 7 et 11 sont assemblées de la manière décrite ci-dessus, les ouvertures 28 réalisées dans les couches 2, 5 et 6 sont alignées les unes avec les autres de manière à former ensemble une ouverture 28 commune traversant ces différentes couches. De même, les ouvertures 29 réalisées dans les couches 2, 5 et 6 sont alignées les unes avec les autres de manière à former ensemble une ouverture 28 commune traversant ces différentes couches. De plus, les découpes 30 réalisées dans les couches 2, 5 et 7 sont également alignées les unes avec les autres de manière à former ensemble une découpe 30 commune traversant ces différentes couches.

Concernant la couche 4, celle-ci présente elle aussi une géométrie globale identique à celle de la couche 2.

Dans cet exemple, la couche 4 est également réalisée en nontissé de polypropylène et a une épaisseur de 410 µm.

La couche 4 se distingue de la couche 2 en ce qu'elle est dépourvue d'ouvertures telles que les ouvertures 28 et 29 de la couche 2 et d'une découpe telle que la découpe 30 de la couche 2.

La couche 12 est une couche de colle formée de deux parties 13 et 14 disposées contre, et donc collées à, la première surface de la couche 3.

En particulier, la partie 13 est configurée pour former une zone de collage au niveau de l'extrémité longitudinale 21 de la couche 3, la partie 14 étant configurée pour former une zone de collage au niveau de l'extrémité longitudinale 22 de la couche 3.

Dans cet exemple, la partie 13 de la couche de colle 12 est recouverte sur sa première surface d'un film de protection (non représenté) du même type que le film de protection 7 et étant lui aussi destiné à être retiré lors de l'utilisation de la housse 1 (voir plus loin ci-dessous).

Dans cet exemple, la deuxième surface de la couche 4 est disposée contre la première surface des couches 12 et 3 et du film de protection recouvrant la première surface de la partie 13 de la couche de colle 12, entraînant un collage uniquement de la deuxième extrémité longitudinale de la couche 4 sur la deuxième extrémité longitudinale de la couche 3 via la partie 14 de la couche de colle 12. La partie 13 de la couche de colle 12 étant pourvue du film de protection, celle-ci n'adhère pas à la première extrémité longitudinale de la couche 4.

A l'état initial, un tel assemblage des différentes couches de la housse 1 confère à celle-ci une épaisseur totale de l'ordre de 1,3 mm, c'est-à-dire une épaisseur totale incluant les protections pelables qui reste faible par rapport aux dimensions longitudinale et latérale de la housse 1.

Une telle housse 1 est de préférence fabriquée par déroulement de bobines de films (non représentées) de manière à former avec chacun de ces films l'une respective des couches décrites ci-dessus. Autrement dit, la housse 1 de l'invention peut être fabriquée à l'aide d'un procédé du type « rouleau à rouleau » (« roll-to-roll » en anglais), qui est une technique de fabrication connue en soi permettant de fabriquer de telles housses en série à cadence très élevée et coût réduit.

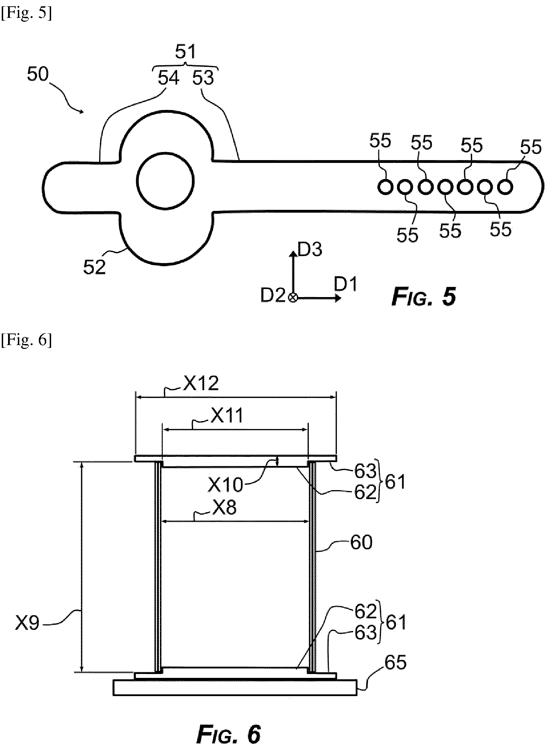

Dans cet exemple, la housse 1 de la figure 1 est prévue pour recevoir un dispositif, tel que le dispositif 50 représenté sur les figures 4 et 5.

Le dispositif 50, ici pris comme exemple non limitatif, est un dispositif médical de thermométrie, destiné à mesurer la température centrale de l'individu de manière non-invasive. Le document WO 2020/249665 A1 décrit des exemples de réalisation d'un dispositif analogue.

En référence aux figures 4 et 5, le dispositif 50 se présente sous la forme d'une nappe 51 allongée le long de la direction D1 et d'un boîtier 52 intégrant notamment des composants électroniques (non représentés) et une batterie (non représentée). La nappe 51 comprend une partie 53 s'étendant longitudinalement d'un premier côté du boîtier 52 et une partie 54 s'étendant longitudinalement de l'autre côté du boîtier 52.

La nappe 51 et le boîtier 52 sont ici surmoulés dans une enveloppe en matériau souple du type silicone, autorisant une déformation de la nappe 51, en particulier en rotation autour d'un axe parallèle à la direction D3.

Dans cet exemple, le dispositif 50 comprend sept capteurs 55 portés par la partie 53 de la nappe 51 et comprenant une extrémité de mesure débouchant transversalement sur une première face de la nappe 51 (voir figure 5). Le dispositif 50 comprend aussi un capteur 56, également porté par la partie 53 de la nappe 51, comprenant une extrémité de mesure débouchant transversalement sur une deuxième face de la nappe 51 (voir figure 4). Le dispositif 50 comprend par ailleurs un capteur 57 porté par le boîtier 52 et débouchant transversalement du boîtier du même côté que le capteur 56 (voir figure 4).

Dans cet exemple, les capteurs 55 sont destinés à mesurer une température cutanée, le capteur 56 est destiné à mesurer une température dans une cavité formée par une aisselle de l'individu et le capteur 57 est destiné à mesurer une température ambiante.

Le dispositif 50 peut aussi comprendre d'autres capteurs, non décrits, par exemple un ou plusieurs capteurs de température, de photopléthysmographie et/ou un ou plusieurs capteurs de mouvements.

Dans cet exemple, le dispositif 50 comprend par ailleurs des moyens d'enregistrement de données de mesure fournies par de tels capteurs et des moyens de communication, par exemple sans fil, permettant de transférer de telles données vers un terminal informatique distant.

Dans cet exemple particulier, la nappe 51 et le boîtier 52 ont une épaisseur, c'est-à-dire une dimension selon la direction D2, d'environ 4 mm et 15 mm, respectivement, le dispositif 50 ayant une masse totale comprise entre 60 et 70 grammes.

En référence aux figures 1 à 5, la housse 1 et le dispositif 50 sont respectivement dimensionnés pour permettre :
- l'introduction de la partie 54 de la nappe 51 du dispositif 50 dans ledit premier espace de la housse 1 via l'ouverture 28 traversant les couches 2, 5 et 6 de la housse 1, et
- l'introduction de la partie 53 de la nappe 51 du dispositif 50 dans ledit deuxième espace de la housse 1 via l'ouverture 29 traversant les couches 2, 5 et 6 de la housse 1,
de sorte que les capteurs 55 du dispositif 50 débouchent de la housse 1 par la découpe 30 traversant les couches 2, 5 et 7 de la housse 1, transversalement vers le bas de la figure 1, et de sorte que le capteur 56 débouche de la housse 1 par la découpe 31 de la couche 3 de la housse 1, transversalement vers le haut de la figure 1.

Dans cet exemple, la housse 1 forme ainsi un espace de réception du dispositif 50 constitué par lesdits premier espace et deuxième espace décrits ci-dessus.

Lors de l'introduction du dispositif 50 dans la housse 1, la housse 1 passe de l'état initial décrit ci-dessus dans lequel les couches 2 et 3 sont plaquées l'une contre l'autre, à un état dit d'utilisation dans lequel le volume de l'espace de réception est augmenté par déformation des couches 2 et 3, sous l'action de l'introduction du dispositif 50 dans la housse 1.

Dans cette configuration, le boîtier 52 du dispositif 50 est positionné sur la deuxième surface de la couche 6, laquelle permet d'éviter de souiller ce dispositif 50 avec la colle de la couche 5. Le capteur 57 est orienté transversalement vers le haut de la figure 1, c'est-à-dire qu'il est en proximité direct de l'échancrure 23 de la couche 3.

Dans l'application ici décrite à titre d'exemple non limitatif, l'ensemble constitué par la housse 1 et le dispositif 50 ainsi introduit dans la housse 1 est disposé sur une surface de peau d'un bras de l'individu, après retrait du film 7, de manière à fixer la housse 1 sur cette surface de peau avec la couche de colle 5.

La pose est réalisée de sorte que les capteurs 55 du dispositif 50 viennent en contact direct avec la peau du bras et de sorte que le capteur 56 soit disposé aux environs de l'aisselle de l'individu.

Dans cet exemple, la couche 4 de la housse 1 est utilisée comme moyen de fixation supplémentaire de manière à former un brassard, afin d'améliorer le maintien en position du dispositif 50 sur le bras de l'individu. Après collage de la housse 1 par la couche de colle 5, la couche 4 est orientée de manière à faire le tour du bras pour venir coller l'extrémité longitudinale 21 de la première surface de la couche 4 sur la partie 13 de la couche de colle 12, initialement pourvue d'un protecteur ou « liner ».

La couche 4 étant ainsi repliée sur elle-même au niveau de son extrémité longitudinale 22, ce collage en situation d'utilisation est donc sollicité en pelage et donc susceptible d'être décollé en cas de surtension. Cela ne pose pas de problème puisque la fonction de la couche 4, lorsqu'elle est présente, est essentiellement de « finir » le périmètre et d'éviter qu'un bord extrême de la housse ne s'accroche et engage un arrachement de la housse. Les inventeurs ont constaté que lorsqu'un cylindre sur lequel la housse est posée se dilate, en particulier par un effort musculaire (type contraction du biceps), la colle résiste néanmoins suffisamment.

Il existe néanmoins des situations d'évolution très significative du diamètre des membres. Il s'agit de certains patients à l'hôpital, qui subissent des défaillances du système cardiovasculaire. Pour compenser la faible pression artérielle, les praticiens peuvent les hyper-perfuser, c'est-à-dire faire passer du sérum physiologique en grande quantité dans l'appareil vasculaire pour faire augmenter la pression artérielle et veineuse. Cela se traduit par un passage de ce liquide dans l'espace extracellulaire. Le corps est alors gonflé d'eau et le diamètre du bras peut significativement évoluer, par exemple de 1 cm. Cette dilatation dure bien sûr longtemps. Les inventeurs ont été surpris de constater que dans cette situation, le collage peut partiellement céder. La conception du collage se comporte alors comme une sorte de fusible permettant de relâcher une part de la tension par pelage. Si le collage fait par exemple 25 mm de long, dans l'axe de la housse, il pourra ainsi relâcher presque 50 mm de longueur au diamètre avant de lâcher, ce qui est très significatif par rapport à la situation décrite. Dans l'exemple de la figure 1, la couche 2 a un comportement très différent de la couche 3. La couche 2 est certes plus épaisse mais surtout c'est elle qui, une fois mise en forme par exemple en cylindre selon un axe transversal, induit la déformation par élongation de la couche 3.

Les inventeurs ont recherché des manières de caractériser ce phénomène et se sont intéressés à la notion de « main » ici utilisée pour comparer leur comportement.

De manière générale, la main d'une couche dépend principalement de l'épaisseur et de la densité de cette couche, ainsi que du sens des fibres et d'éventuels effets de calandrage.

Pour une couche telle qu'une feuille de papier conventionnelle comprenant des fibres végétales, la main peut être exprimée comme le rapport entre l'épaisseur de la feuille et son grammage. Concernant cependant une structure telle que les couches 2 et 3 de la housse 1 de l'invention, qui comprennent dans cet exemple un matériau nontissé comportant des fibres plastiques, cette approximation ne donne pas de représentation suffisamment fiable du comportement du matériau et ne permet pas de le comparer de manière réaliste.

Les inventeurs se sont alors intéressés à la notion de « drapé », définie dans la norme « ISO 9073-9:2008 », le drapé exprimant l'aptitude d'une éprouvette d'étoffe à se déformer de son propre poids. Cette notion très utilisée pour comparer le « tombé » de tissus est néanmoins trop limitative en l'espèce puisque les tensions en présence dans la housse 1 une fois posée sur le corps peuvent être incomparablement plus importantes que les tensions en présence dans un drapé.

Les inventeurs proposent ainsi une nouvelle méthode de caractérisation du comportement des couches 2 et 3 visant à tenir compte de leurs sollicitations effectives lors de l'utilisation de la housse 1. Avec cette méthode, on mesure la masse déclenchant un affaissement d'un cylindre 60 (voir figure 6) constitué par enroulement de la matière utilisée pour former ces couches. Il est ici proposé d'appeler ce paramètre « résistance cylindrique ».

Dans un exemple de mise en œuvre de cette méthode, illustré sur la figure 6, le cylindre 60 est formé par enroulement d'une couche de matière en deux tours de manière à former un cylindre 60 ayant un diamètre X8 de 40 mm et une longueur X9 de 38 mm. Le cylindre 60 ou rouleau ainsi formé est maintenu sous cette forme à l'aide d'un adhésif double-face ayant dans cet exemple une largeur de 10 mm et s'étendant sur toute la longueur X9 du cylindre 60 de manière à fixer une extrémité libre de la couche de matière formant le cylindre à une surface formée par l'une des spires de ce cylindre 60. Des disques 61 de plastique rigides d'épaisseur X10 égale à 6 mm sont assemblés de manière concentrique, chaque disque 61 comprenant une portion interne 62 ayant un diamètre X11 de 39 mm et une portion externe 63 ayant un diamètre X12 de 50 mm et formant un épaulement par rapport à la portion interne 62. L'un des disques 61, représenté en bas sur la figure 6 et appelé « disque inférieur », est placé sur un plateau 65 d'une balance et le cylindre 60 est disposé sur ce disque inférieur 61 de sorte que sa portion interne 62 s'étende radialement dans le cylindre 60 au niveau de l'une de ses extrémités, cette dernière venant en appui sur l'épaulement formé par la portion externe 63 du disque inférieur 61. La balance est tarée avec le cylindre 60 et le disque inférieur 61 ainsi disposés sur le plateau 65. L'autre disque 61, représenté en haut sur la figure 6 et appelé « disque supérieur », est ensuite disposé de manière symétrique sur l'extrémité opposée du cylindre 60, de la manière illustrée sur la figure 6. Une telle disposition des disques 61 par rapport au cylindre 60, en particulier le fait de loger la portion interne 62 des disques 61 dans les extrémités du cylindre 60, permet d'éviter un glissement, radialement vers l'intérieur, des extrémités du cylindre 60 et d'éviter ainsi un écrasement prématuré du cylindre 60.

Dans cette configuration, le cylindre 60 est progressivement chargé par application d'un effort mécanique ayant pour principale composante une force dirigée selon la longueur du cylindre 60, en l'occurrence du haut vers le bas de la figure 6. Un tel effort peut être généré par remplissage d'un réservoir (non représenté) disposé sur le disque supérieur 61 à l'aide d'un liquide, en prenant soin de bien centrer le réservoir par rapport au cylindre 60. L'effort est appliqué de manière progressive sur le cylindre 60, par exemple de 20 grammes par seconde, jusqu'à effondrement du cylindre 60.

L'opération qui vient d'être décrite est de préférence réalisée plusieurs fois avec plusieurs échantillons de matière similaires, par exemple cinq fois. On retiendra alors comme valeur de résistance cylindrique la moyenne des valeurs de la série, par exemple après avoir exclu la valeur la plus haute et la valeur la plus basse.

La housse 1 de l'invention permet notamment d'éviter ou de réduire le risque de formation de plis de la couche 2 et/ou d'éviter des phénomènes de décollement, de glissement ou encore de roulement de la housse 1 sur la surface de peau.

Plus généralement, la housse 1 de l'invention permet de plaquer un dispositif tel que le dispositif 50 sur la surface de la peau et de maintenir un positionnement précis de ce dispositif, y compris en cas de sollicitations de la housse 1 et/ou du dispositif 50 sous l'action de mouvements du corps. Il est ainsi possible de réaliser des mesures fiables, par exemple en continu sur des périodes de plusieurs heures ou jours.

Bien entendu, de nombreuses variantes peuvent être apportées à la description qui précède sans sortir du cadre de l'invention. Par exemple, la housse de l'invention peut être configurée pour recevoir un dispositif différent du dispositif 50 décrit ci-dessus, notamment un dispositif de mesure incluant d'autres types de capteurs, par exemple des capteurs thermiques, et/ou comportant des organes destinés par exemple à diffuser un principe actif sous forme liquide, tels que des microaiguilles, un réservoir et/ou une micro-pompe. En outre, la housse 1 peut être configurée pour être disposée sur des parties de corps différentes du bras, par exemple sur le torse ou d'autres parties du corps présentant une courbure importante.

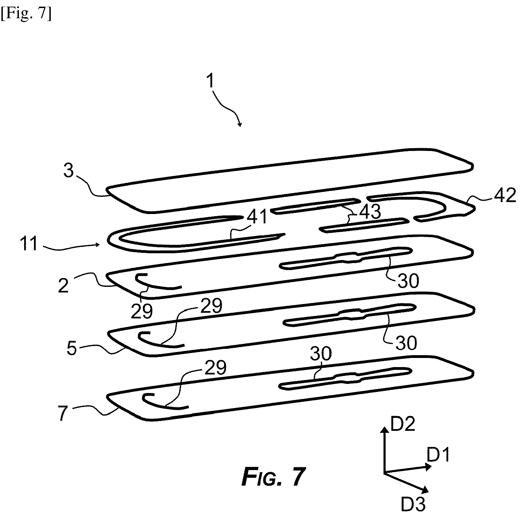

En fonction notamment du type de dispositif qu'elle est destinée à recevoir et de la partie du corps contre lequel elle est destinée à maintenir un tel dispositif, la housse de l'invention peut présenter des caractéristiques structurelles et/ou géométriques différentes de celles décrites ci-dessus en référence à la figure 1. Par exemple, la housse peut être dépourvue des couches 4 et 12 représentées sur la figure 1 et/ou peut former un espace de réception unitaire, par exemple tel qu'illustré de manière non limitative sur la figure 7.

Dans le mode de réalisation de la figure 7, la housse 1 se distingue de celle de la figure 1 en ce qu'elle ne comprend pas de parties 23 et 26, ni de couches 4 et 6, ni de découpe 31 dans la couche 3, la couche 7 comportant une ouverture 29 alignées avec les autres ouvertures 29 pour permettre une insertion dans la housse 1 d'un dispositif avant retrait de la couche 7 et collage de l'ensemble par la couche de colle 5. La description qui précède s'applique par analogie à ce mode de réalisation, qui peut par exemple être utilisé pour recevoir un dispositif destiné à diffuser un principe actif via la découpe 30.

Dans une variante de réalisation, non représentée, la housse se distingue de celle de la figure 7 en ce qu'elle est dépourvue des ouvertures 29 et en ce que la couche de colle 11 est configurée pour ne pas coller les couches 2 et 3 l'une à l'autre par l'une de leurs extrémités longitudinales. A cet effet, une portion de la partie 41 de la couche de colle 11 peut être supprimée. Les couches 2 et 3 peuvent ainsi définir entre elles une ouverture au niveau d'une extrémité longitudinale de la housse 1, constituée par une partie non collée des couches 2 et 3 et permettant une insertion du dispositif entre celles-ci sans qu'il soit nécessaire de réaliser des ouvertures telles que les ouvertures 29.

Pour autre exemple, des matériaux différents de ceux décrits ci-dessus peuvent être utilisés pour une ou plusieurs couches de la housse 1. Par exemple, la couche 3 peut comprendre un matériau susceptible de déformation plastique.

La structure de la housse 1 des différents modes de réalisation décrits est une structure en couches empilées, qui présente l'avantage de pouvoir être fabriquée à l'aide d'un procédé du type rouleau à rouleau. Bien entendu, la housse 1 peut néanmoins être fabriquée en utilisant d'autres techniques. D'autres techniques de fabrication peuvent par ailleurs permettent d'obtenir certains avantages de la housse 1 de l'invention avec des caractéristiques structurelles différentes. Ainsi, il est notamment possible de réduire le risque de formation de plis de la couche 2 en réalisant une couche 3 plissée, par exemple à l'aide d'un procédé de collage sous tension, d'extension de matériau à force ou par cuisson, de rétractation de matériau de la couche 2, ou encore un thermoformage.

## Revendications

1. Housse (1) destinée à maintenir un dispositif (50) médical et/ou de mesure de données physiologiques et/ou de diffusion d'un principe actif et/ou de prélèvement d'un fluide corporel à proximité de, ou contre, une surface de peau d'un individu, cette housse (1) comprenant une première couche (2) et une deuxième couche (3) reliées l'une à l'autre de manière à être plaquées l'une contre l'autre lorsque la housse (1) est dans un état initial, la housse (1) étant configurée pour autoriser une déformation de la deuxième couche (3) faisant passer la housse (1) de l'état initial à un état d'utilisation dans lequel la première couche (2) et la deuxième couche (3) définissent entre elles un espace de réception apte à recevoir ledit dispositif (50), la housse (1) comprenant une ouverture (28, 29) configurée pour permettre une introduction du dispositif (50) dans l'espace de réception, la première couche (2) comprenant une surface externe pourvue d'un matériau adhésif tel qu'une colle silicone ou acrylate permettant de fixer la housse (1) sur ladite surface de peau, **caractérisée en ce que** la première couche (2) a un coefficient de drapé supérieur à celui de la deuxième couche (3).

2. Housse (1) selon la revendication 1, dans laquelle la première couche (2) et la deuxième couche (3) comprennent chacune une surface interne et sont reliées l'une à l'autre par collage ou soudage de parties de leur surface interne.

3. Housse (1) selon la revendication 1 ou 2, dans laquelle la première couche (2) et/ou la deuxième couche (3) comprennent un matériau nontissé, par exemple un matériau plastique ou élastomère, et/ou un textile.

4. Housse (1) selon l'une quelconque des revendications 1 à 3, dans laquelle la deuxième couche (3) est extensible ou élastique, et/ou autorisant une déformation plastique ou élastique de la deuxième couche (3) plus importante que la première couche (2).

5. Housse (1) selon l'une quelconque des revendications 1 à 4, dans laquelle la première couche (2) a une épaisseur supérieure, par exemple plus de deux fois supérieure, à une épaisseur de la deuxième couche (3) et/ou dans laquelle la première couche (2) a un coefficient de drapé plus de deux fois supérieur, à celui de la deuxième couche (3).

6. Housse (1) selon l'une quelconque des revendications 1 à 5, dans laquelle la première couche (2) comprend une découpe (30) autorisant un contact direct d'une partie (55) du dispositif (50) avec la surface de peau lorsque le dispositif (50) est reçu dans l'espace de réception.

7. Housse (1) selon l'une quelconque des revendications 1 à 6, comprenant une troisième couche (4) ayant une première partie reliée à la première couche (2) et/ou à la deuxième couche (3) et une deuxième partie configurée pour être collée sur ladite surface de peau et/ou sur une partie de la housse (1) et/ou sur une partie du dispositif (50), par exemple de manière à former un brassard.

8. Ensemble comprenant une housse (1) selon l'une quelconque des revendications 1 à 7 ainsi qu'un dispositif (50) médical et/ou de mesure de données physiologiques et/ou de diffusion d'un principe actif et/ou de prélèvement d'un fluide corporel tel qu'un dispositif médical de thermométrie.

9. Procédé de fabrication d'une housse (1) selon l'une quelconque des revendications 1 à 7, comprenant une étape de déroulement d'une ou plusieurs bobines de film de manière à former la première couche (2) et/ou la deuxième couche (3) et une étape d'assemblage de la première couche (2) avec la deuxième couche (3), l'étape d'assemblage comprenant de préférence un déroulement d'une ou plusieurs bobines d'un film adhésif, notamment un film adhésif double face.

10. Procédé de pose d'une housse (1) selon l'une quelconque des revendications 1 à 7 sur une surface de peau d'un individu, comprenant une étape d'application de la première couche (2) sur la surface de peau.

## Patentansprüche

1. Hülle (1) zum Halten einer medizinischen Vorrichtung (50) und/oder einer Vorrichtung zur Messung physiologischer Daten und/oder zur Abgabe eines Wirkstoffs und/oder zur Entnahme einer Körperflüssigkeit in der Nähe von oder direkt auf einer Hautoberfläche einer Person, wobei diese Hülle (1) eine erste Schicht (2) und eine zweite Schicht (3) umfasst, die so miteinander verbunden sind, dass sie in einem Ausgangszustand der Hülle (1) aneinander gedrückt sind, wobei die Hülle (1) so ausgebildet ist, dass sie eine Verformung der zweiten Schicht (3) ermöglicht, wodurch die Hülle (1) vom Ausgangszustand in einen Gebrauchszustand übergeht, in dem die erste Schicht (2) und die zweite Schicht (3) zwischen sich einen Aufnahmeraum bilden, der zur Aufnahme der Vorrichtung (50) geeignet ist, wobei die Hülle (1) eine Öffnung (28, 29) aufweist, die so ausgebildet ist, dass sie ein Einführen der Vorrichtung (50) in den Aufnahmeraum ermöglicht, wobei die erste Schicht (2) eine Außenfläche aufweist, die mit einem Klebematerial wie beispielsweise einem Silikon- oder Acrylatkleber versehen ist, das es ermöglicht, die Hülle (1) auf der Hautoberfläche zu befestigen,
**dadurch gekennzeichnet, dass** die erste Schicht (2) einen höheren Drape-Koeffizienten als die zweite Schicht (3) aufweist.

2. Hülle (1) nach Anspruch 1, wobei die erste Schicht (2) und die zweite Schicht (3) jeweils eine Innenfläche aufweisen und durch Verkleben oder Verschweißen von Teilen ihrer Innenfläche miteinander verbunden sind.

3. Hülle (1) nach Anspruch 1 oder 2, wobei die erste Schicht (2) und/oder die zweite Schicht (3) ein Vliesmaterial, beispielsweise ein Kunststoff- oder Elastomermaterial, und/oder ein Textilmaterial aufweist.

4. Hülle (1) nach einem der Ansprüche 1 bis 3, wobei die zweite Schicht (3) dehnbar oder elastisch ist und/oder eine größere plastische oder elastische Verformung der zweiten Schicht (3) als der ersten Schicht (2) ermöglicht.

5. Hülle (1) nach einem der Ansprüche 1 bis 4, wobei die erste Schicht (2) eine Dicke aufweist, die größer ist, beispielsweise mehr als doppelt so groß ist wie eine Dicke der zweiten Schicht (3), und/oder wobei die erste Schicht (2) einen Drape-Koeffizienten aufweist, der mehr als doppelt so groß ist wie der der zweiten Schicht (3).

6. Hülle (1) nach einem der Ansprüche 1 bis 5, wobei die erste Schicht (2) einen Ausschnitt (30) aufweist, der einen direkten Kontakt eines Teils (55) der Vorrichtung (50) mit der Hautoberfläche ermöglicht, wenn die Vorrichtung (50) in dem Aufnahmeraum aufgenommen ist.

7. Hülle (1) nach einem der Ansprüche 1 bis 6, die eine dritte Schicht (4) mit einem ersten Teil, der mit der ersten Schicht (2) und/oder der zweiten Schicht (3) verbunden ist, und einen zweiten Teil umfasst, der so ausgebildet ist, dass er auf die Hautoberfläche und/oder auf einen Teil der Hülle (1) und/oder auf einen Teil der Vorrichtung (50) geklebt werden kann, beispielsweise um eine Manschette zu bilden.

8. Baugruppe, die eine Hülle (1) nach einem der Ansprüche 1 bis 7 sowie eine medizinische Vorrichtung (50) und/oder eine Vorrichtung zur Messung physiologischer Daten und/oder zur Abgabe eines Wirkstoffs und/oder zur Entnahme einer Körperflüssigkeit, wie beispielsweise eine medizinische Thermometervorrichtung, umfasst.

9. Verfahren zur Herstellung einer Hülle (1) nach einem der Ansprüche 1 bis 7, das einen Schritt zum Abwickeln einer oder mehrerer Folienrollen umfasst, um die erste Schicht (2) und/oder die zweite Schicht (3) zu bilden, sowie einen Schritt zum Verbinden der ersten Schicht (2) mit der zweiten Schicht (3), wobei der Verbindungsschritt vorzugsweise das Abwickeln einer oder mehrerer Rollen einer Klebefolie, insbesondere einer doppelseitigen Klebefolie, umfasst.

10. Verfahren zum Anbringen einer Hülle (1) nach einem der Ansprüche 1 bis 7 auf einer Hautoberfläche einer Person, das einen Schritt zum Aufbringen der ersten Schicht (2) auf die Hautoberfläche umfasst.

## Claims

1. A cover (1) for holding a medical device (50) and/or device for measuring physiological data and/or for diffusing an active ingredient and/or for sampling a body fluid in the vicinity of, or against, a skin surface of an individual, this cover (1) comprising a first layer (2) and a second layer (3) connected to each other so as to be pressed against each other when the cover (1) is in an initial state, the cover (1) being configured to allow a deformation of the second layer (3) moving the cover (1) from the initial state to a state of use wherein the first layer (2) and the second layer (3) define between them a receiving space suitable for receiving said device (50), the cover (1) comprising an opening (28, 29) configured to allow the device (50) to be inserted into the receiving space, the first layer (2) comprising an outer surface provided with an adhesive material such as a silicone or acrylate adhesive enabling the cover (1) to be attached to said skin surface, **characterized in that** the first layer (2) has a drape coefficient greater than that of the second layer (3).

2. The cover (1) according to claim 1, wherein the first layer (2) and the second layer (3) each comprise an inner surface and are connected to each other by bonding or welding parts of their inner surface.

3. The cover (1) according to claim 1 or 2, wherein the first layer (2) and/or the second layer (3) comprise a nonwoven material, for example a plastic or elastomer material, and/or a textile.

4. The cover (1) according to any one of claims 1 to 3, wherein the second layer (3) is stretchable or elastic, and/or allows greater plastic or elastic deformation of the second layer (3) than the first layer (2).

5. The cover (1) according to any one of claims 1 to 4, wherein the first layer (2) has a thickness greater than, for example more than twice, a thickness of the second layer (3) and/or wherein the first layer (2) has a drape coefficient more than twice, that of the second layer (3).

6. The cover (1) according to any one of claims 1 to 5, wherein the first layer (2) comprises a cutout (30) allowing direct contact of a part (55) of the device (50) with the skin surface when the device (50) is received in the receiving space.

7. The cover (1) according to any one of claims 1 to 6, comprising a third layer (4) having a first part connected to the first layer (2) and/or to the second layer (3) and a second part configured to be bonded to said skin surface and/or to a part of the cover (1) and/or to a part of the device (50), for example so as to form a cuff.

8. An assembly comprising a cover (1) according to any one of claims 1 to 7 as well as a medical device (50) and/or device for measuring physiological data and/or for diffusing an active principle and/or for sampling a body fluid, such as a medical thermometry device.

9. A method of manufacturing a cover (1) according to any one of claims 1 to 7, comprising a step of unwinding one or more reels of film so as to form the first layer (2) and/or the second layer (3) and a step of assembling the first layer (2) with the second layer (3), the assembly step preferably comprising unwinding one or more reels of an adhesive film, in particular a double-sided adhesive film.

10. A method of applying a cover (1) according to any one of claims 1 to 7 to a skin surface of an individual, comprising a step of applying the first layer (2) to the skin surface.
